(19) Europäisches Patentamt
European Patent Office
Office européen des brevets

(11) Veröffentlichungsnummer: **0 055 484**
**A1**

# (12) EUROPÄISCHE PATENTANMELDUNG

(21) Anmeldenummer: 81110821.6

(22) Anmeldetag: 29.12.81

(51) Int. Cl.³: C 07 D 273/04
A 61 K 31/535

(30) Priorität: 31.12.80 HU 316580

(43) Veröffentlichungstag der Anmeldung:
07.07.82 Patentblatt 82/27

(84) Benannte Vertragsstaaten:
BE CH DE FR GB IT LI NL SE

(71) Anmelder: RICHTER GEDEON VEGYESZETI GYAR RT
Gyömröi ut 21
H-1103 Budapest(HU)

(72) Erfinder: Ürögdi, László, Dipl. Chem.-Ing.
Névtelen u. 35
H-1163 Budapest(HU)

(72) Erfinder: Patthy, Agnes geb. Lukáts
Fenyö u. 10
H-1016 Budapest(HU)

(72) Erfinder: Kisfaludy, Lajos, Dr. Dipl. Chem.-Ing.
Rlado u. 6/a
H-1026 Budapest(HU)

(72) Erfinder: Moravcsik, Erno, Dipl.-Chem.
Lágymányosi u. 28
H-1111 Budapest(HU)

(72) Erfinder: Tüdos, Helga, Dr. geb. Feuer Dipl.-Chem.
Felhö u. 18
H-1125 Budapest(HU)

(72) Erfinder: Ötvös, Lászlo, Dr. Dipl.-Chem.
Boroka u. 13
H-1025 Budapest(HU)

(72) Erfinder: Tegyei, Zsuzsanna, Dr. Dipl.-Chem.
Györi u. 2/a
H-1123 Budapest(HU)

(72) Erfinder: Pálosi, Eva, Dr.
Vend u. 21
H-1025 Budapest(HU)

(72) Erfinder: Sarkadi, Adám, Dr.
Thököly u. 85
H-1143 Budapest(HU)

(72) Erfinder: Szporny, Lászlo, Dr.
Szabolcska M. u. 7
H-1114 Budapest(HU)

(74) Vertreter: Beszédes, Stephan G. Dr.
Münchener Strasse 80a Postfach 1168
D-8060 Dachau(DE)

## (54) Tetrahydro-1,2,4-oxadiazin-5-onderivate, Verfahren zu ihrer Herstellung und diese Verbindungen enthaltende Arzneimittel.

(57) Tetrahydro-1,2,4-oxadiazin-5-onderivate der allgemeinen Formel

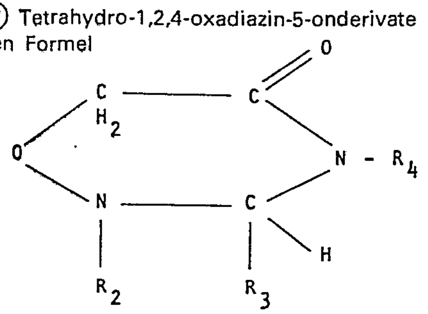

1 ,

sowie Verfahren zu ihrer Herstellung.

Diese Verbindungen können in Arzneimitteln auf dem Gebiet der Therapie, insbesondere gegen Erkrankungen des Zentralnervensystemes, verwendet werden.

EP 0 055 484 A1

Croydon Printing Company Ltd.

Die Erfindung betrifft neue Tetrahydro-1,2,4-oxadiazin-5-onderivate, ein Verfahren zu ihrer Herstellung und diese Verbindungen enthaltende Arzneimittel, insbesondere solche mit Wirkungen auf das Zentralnervensystem, vor allem krampfhemmenden beziehungsweise antikonvulsiven Wirkungen.

Das Tetrahydro-1,2,4-oxadiazin und einige substituierte Derivate desselben wurden erst in den letzteren Jahren bekannt. Ihre Synthese wurde zuerst von Calgano und Mitarbeitern (J. Org. Chem. 39 [1974], 162) beschrieben; von diesen Verfassern wurden durch die Cycloaddition von 1-Aroylaziridinen an Nitrone die entsprechenden 4-Aroyltetrahydro-1,2,4-oxadiazine erhalten. Später wurden von F. G. Riddel und Mitarbeitern (Heterocycles 9 [1978], 267; Tetrahedron 35 [1979], 1 391) durch Kondensation von N-(Alkyl)-O-(methylaminoäthyl)-hydroxylaminen mit Formaldehyd Tetrahydro-1,2,4-oxadiazine hergestellt. Über eine etwaige biologische Wirksamkeit dieser Produkte ist jedoch im Schrifttum nichts erwähnt und es wurden bisher auch keine in der 5-Stellung eine Oxogruppe aufweisenden Tetrahydro-1,2,4-oxadiazinderivate beschrieben.

Der Erfindung liegt die Aufgabe zugrunde, überlegene pharmakologische Wirkungen aufweisende neue Tetrahydro-1,2,4-oxadiazin-5-onderivate, ein Verfahren zur Herstellung derselben und diese Verbindungen enthaltende Arzneimittel zu schaffen.

Das Obige wurde überraschenderweise durch die Erfindung erreicht.

Es wurde nämlich überraschenderweise festgestellt, daß die im folgenden festgelegten substituierten Tetrahydro-1,2,4-oxadiazin-5-one überlegene wertvolle pharmakologische Eigenschaften, insbesondere vorteilhafte Wirkungen auf das Zentralnervensystem, vor allem krampfhemmende beziehungsweise antikonvulsive Wirkungen, haben.

Gegenstand der Erfindung sind daher Tetrahydro-1,2,4-oxadiazin-5-onderivate der allgemeinen Formel

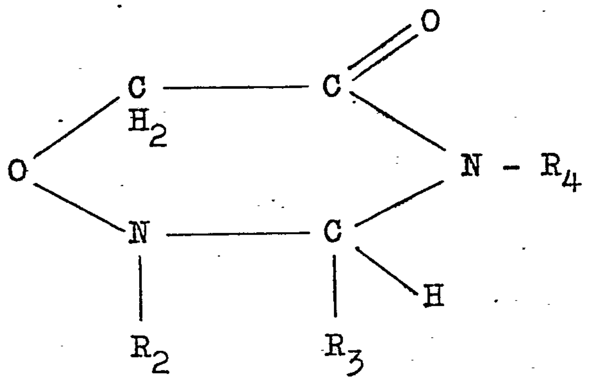

I ,

worin

$R_2$ für einen Benzyloxycarbonylrest oder einen Alkylcarbonylrest mit 1 bis 4 Kohlenstoffatom(en) im Alkylteil steht,

$R_3$ einen, gegebenenfalls durch 1 bis 3 Alkoxyrest(e) mit 1 bis 4 Kohlenstoffatom(en) substituierten, Phenylrest bedeutet und

$R_4$ einen Alkyl- oder Cycloalkylrest mit 1 bis 11 Kohlenstoffatom(en), einen Hydroxymethylrest, einen Halogenmethylrest, einen Alkylcarbonyloxymethylrest mit 1 bis 4 Kohlenstoffatom(en) im Alkylteil oder einen, gegebenenfalls durch 1 bis 3 Alkoxyrest(e) mit 1 bis 4 Kohlenstoffatom(en) substituierten, Benzoyloxymethylrest darstellt.

Vorzugsweise ist der Alkylcarbonylrest, für den $R_2$ stehen kann, ein solcher mit 1 oder 2 Kohlenstoffatom(en) im Alkylteil, insbesondere ein Acetylrest.

Es ist auch bevorzugt, daß der beziehungsweise die Alkoxyrest(e), durch welchen beziehungsweise welche der Phenylrest, für den $R_3$ stehen kann, substituiert sein kann, ein solcher beziehungsweise solche mit 1 oder 2, insbesondere 1, Kohlenstoffatom(en) ist beziehungsweise sind.

Ferner ist es bevorzugt, daß der Alkylrest, für den $R_4$ stehen kann, ein solcher mit 1 bis 8, insbesondere 1 bis 6, ganz besonders 1 bis 3, vor allem 1 oder 2, Kohlenstoffatom(en) ist.

Weiterhin ist es bevorzugt, daß der Cycloalkylrest, für den $R_4$ stehen kann, ein solcher mit 3 bis 8, insbesondere 3 bis 6, ganz besonders 3 oder 6, Kohlenstoffatomen ist.

Vorzugsweise ist der Halogenmethylrest, für den $R_4$ stehen kann, ein Chlormethyl- oder Brommethylrest, insbesondere ein Chlormethylrest.

Es ist auch bevorzugt, daß der Alkylcarbonyloxymethylrest, für den $R_4$ stehen kann, ein solcher mit 1 oder 2, insbesondere 1, Kohlenstoffatom(en) im Alkylteil ist.

Ferner ist es bevorzugt, daß der beziehungsweise die Alkoxyrest(e), durch welchen beziehungsweise welche der Benzoyloxymethylrest, für den $R_4$ stehen kann, substituiert sein kann, ein solcher beziehungsweise solche mit 1 oder 2, insbesondere 1, Kohlenstoffatom(en) ist beziehungsweise sind.

Besonders bevorzugte erfindungsgemäße Verbindungen sind 2-Acetyl-3-phenyl-4-(hydroxymethyl)-tetrahydro-1,2,4-oxadiazin-5-on, 2-Acetyl-3-phenyl-4-(acetoxymethyl)-tetrahydro-1,2,4-oxadiazin-5-on und 2-Acetyl-3-phenyl-4-(benzoyloxymethyl)-tetrahydro-1,2,4-oxadiazin-5-on.

Gegenstand der Erfindung ist auch ein Verfahren zur Herstellung der erfindungsgemäßen Verbindungen, welches dadurch gekennzeichnet ist, daß

a) zur Herstellung von Tetrahydro-1,2,4-oxadiazin-5-onderivaten der allgemeinen Formel I, bei welchen $R_4$ einen Alkyl- oder Cycloalkylrest mit 1 bis 11 Kohlenstoffatom(en) darstellt, α-Aminooxycarbonsäureamide der allgemeinen Formel

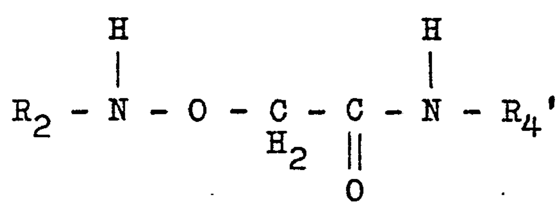

II ,

worin

$R_4'$ einen Alkyl- oder Cycloalkylrest mit 1 bis 11 Kohlenstoffatom(en) bedeutet und

$R_2$ wie oben festgelegt ist,

in aprotonischen (aprotischen) oder protonischen (protischen) Medien in Gegenwart von Säuren mit, gegebenenfalls am Ring durch 1 bis 3 Alkoxy-

rest(e) mit 1 bis 4 Kohlenstoffatom(en) substituiertem, Benzaldehyd der allgemeinen Formel

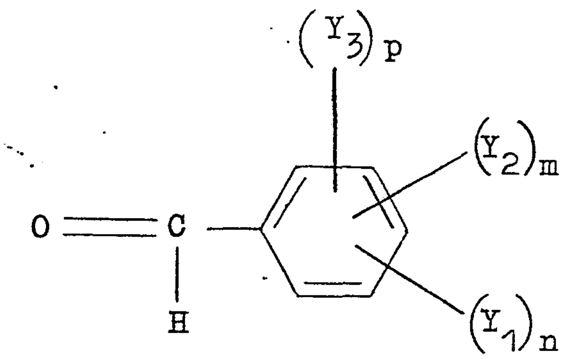

III ,

worin

| | |
|---|---|
| $Y_1$ , $Y_2$ und $Y_3$ | für Alkoxyreste mit 1 bis 4 Kohlenstoffatom(en) stehen und |
| n, m und p | 0 oder 1 sind, |

kondensiert werden oder

b) zur Herstellung der Tetrahydro-1,2,4-oxadiazin-5-onderivate der allgemeinen Formel I, bei welchen $R_4$ einen Alkyl- oder Cycloalkylrest mit 1 bis 11 Kohlenstoffatom(en) bedeutet, Tetrahydro-1,2,4-oxadiazin-5-onderivate der allgemeinen Formel

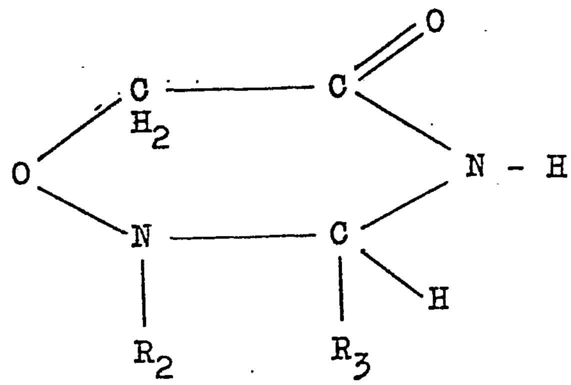

IV ,

worin $R_2$ und $R_3$ wie oben festgelegt sind, in Gegenwart von Basen mit Verbindungen der allgemeinen Formel

$$X - R_4' \qquad V ,$$

worin

X für einen reaktionsfähigen Esterrest, insbesondere ein Halogenatom, ganz besonders ein Chloratom, steht und

$R_4'$ wie oben festgelegt ist,

umgesetzt werden oder

c) zur Herstellung der Tetrahydro-1,2-4-oxadiazin-5--onderivate der allgemeinen Formel I, bei welchen $R_4$ für einen Hydroxymethylrest, einen Halogenmethylrest, einen Alkylcarbonyloxymethylrest mit 1 bis 4 Kohlenstoffatom(en) im Alkylteil oder einen, gegebenenfalls durch 1 bis 3 Alkoxyrest(e) mit 1 bis 4 Kohlenstoffatom(en) substituierten, Benzoyloxymethylrest steht, Tetrahydro-1,2,4-oxadiazin-5-onderivate der allgemeinen Formel

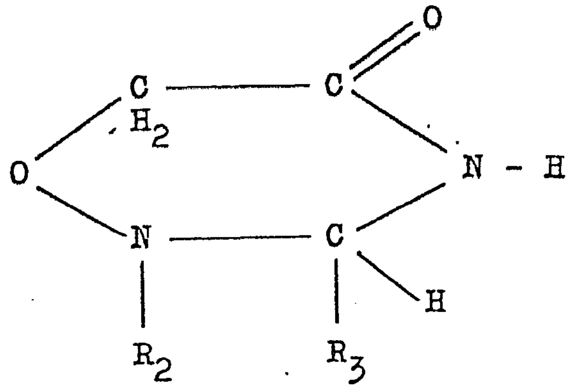

IV ,

worin $R_2$ und $R_3$ wie oben festgelegt sind, in Gegenwart von Basen, insbesondere tertiären organischen Basen, ganz besonders tertiären Aminen, mit Formaldehyd umgesetzt werden und gegebenenfalls die erhaltenen Tetrahydro-1,2,4-oxadiazin-5-onderivate der allgemeinen Formel I, bei welchen $R_4$ für einen Hydroxymethylrest steht, durch Behandeln mit Halogenierungsmitteln, insbesondere Thionylhalogeniden, beziehungsweise passenden Acylierungsmitteln, insbesondere Carbonsäurehalogeniden oder Carbonsäureanhydriden, in die entsprechenden Tetrahydro-1,2,4-oxadiazin-5-onderivate der allgemeinen Formel I, bei welchen $R_4$ für einen Halogenmethylrest beziehungsweise einen Alkylcarbonyloxymethylrest mit 1 bis 4 Kohlenstoffatom(en) im Alkylteil oder einen, gegebenenfalls durch 1 bis 3 Alkoxyrest(e) mit 1 bis 4 Kohlenstoffatom(en) substituierten, Benzoyloxymethylrest steht, überführt werden,

worauf gegebenenfalls nach einer der obigen Varianten erhaltene Tetrahydro-1,2,4-oxadiazin-5-onderivate der allgemeinen Formel I, bei welchen $R_2$ für einen Benzyloxycarbonylrest steht, zweckmäßig durch deren katalytisches Hydrieren und Behandeln mit den passenden Acylierungsmitteln, insbesondere Acetylierungsmitteln, in die entsprechenden Tetrahydro-1,2,4-oxadiazin-5-onderivate der

allgemeinen Formel I, bei welchen $R_2$ für einen Alkylcarbonylrest mit 1 bis 4 Kohlenstoffatom(en) im Alkylteil steht, überführt werden.

Als aprotonische Medien für die Umsetzung der Variante a) des erfindungsgemäßen Verfahrens können zweckmäßig Kohlenwasserstoffe, vorzugsweise aromatische Kohlenwasserstoffe, insbesondere Benzol und/oder Toluol, verwendet werden. Zur Erhöhung der Löslichkeit der umzusetzenden Verbindungen können solche Lösungsmittel nach Bedarf mit einem Gehalt an kleineren bis gleichen Mengen von Butylacetat eingesetzt werden. Als die Umsetzung katalysierende Säuren können zum Beispiel Mineralsäuren, wie Schwefelsäure, und, vor allem im Falle der Verwendung von gegen Säuren empfindlichen Ausgangsstoffen, besonders vorteilhaft Campher-10-sulfonsäure, verwendet werden (im Schrifttum wurde die Verwendung von Campher-10-sulfonsäure für solche Zwecke noch nicht erwähnt). Die Umsetzung wird zweckmäßig bei der Siedetemperatur des Reaktionsgemisches durchgeführt und das entstandene Wasser kann mit Hilfe eines entsprechenden Aufsatzes laufend aus dem Reaktionsgemisch entfernt werden.

Als protonische Medien für die Umsetzung der Variante a) des erfindungsgemäßen Verfahrens können vorteilhaft Gemische von Essigsäure und Essigsäureanhydrid, zum Beispiel ein Gemisch von Essigsäure und Essigsäureanhydrid im Volumverhältnis von 1 : 1, verwendet werden. Auch in diesem Fall können als die Umsetzung katalysierende Säuren zum Beispiel Mineralsäuren oder Campher-10-sulfonsäure in geringen Mengen (etwa 5 bis 8 Gew.-% des Lösungsmittels) verwendet werden. Bei Verwendung von protonischen Medien kann die Umsetzung mit gutem Erfolg bei Raumtemperatur (etwa 15 bis 25°C) durchgeführt werden.

In beiden Fällen, das heißt sowohl in aprotonischen als auch in protonischen Medien dauert der vollständige Reaktionsablauf ziemlich lange, wobei dazu im allgemeinen meistens einige Tage erforderlich sind. Daher ist es zweckmäßig, den Verlauf der Reaktion durch Dünnschichtchromatographie zu verfolgen. Nach dem Ende der Reaktion kann das Lösungsmittel auf die übliche Weise entfernt, der Rückstand in einem mit Wasser nicht mischbaren Lösungsmittel gelöst und die Lösung mit einer verdünnten wäßrigen Säurebeziehungsweise Alkalilösung gewaschen, getrocknet und eingedampft werden. Das als Rückstand erhaltene Endprodukt kann gegebenenfalls durch Umkristallisieren gereinigt werden.

Das Umsetzen der Tetrahydro-1,2,4-oxadiazin-5-onderivate der allgemeinen Formel IV, bei welchen es sich um am Stickstoffatom in der 4-Stellung nicht substituierte Verbindungen handelt, mit den Verbindungen der allgemeinen Formel V nach der Variante b) des erfindungsgemäßen Verfahrens kann unter den üblichen Bedingungen der bekannten N-Alkylierungsreaktionen durchgeführt werden. Als Alkylierungsmittel können vorteilhaft den einzuführenden Alkylrest aufweisende Alkylhalogenide in Gegenwart von säurebindenden Mitteln eingesetzt werden.

Zur Umsetzung der Tetrahydro-1,2,4-oxadiazin-5-onderivate der allgemeinen Formel IV mit dem Formaldehyd nach der Variante c) des erfindungsgemäßen Verfahrens zum Einführen eines Hydroxymethylrestes in die 4-Stellung des Oxadiazinringes wird der Formaldehyd vorteilhaft in Form einer wäßrigen Formaldehydlösung eingesetzt. Als tertiäres organisches Amin wird für diese Umsetzung vorteilhaft Triäthylamin verwendet.

Die Umsetzung der so hergestellten 4-(Hydroxymethyl)-tetrahydro-1,2,4-oxadiazinderivate, welche sehr vorteilhafte Ausgangsverbindungen zur Herstellung von weiteren 4-substituierten Tetrahydro-1,2,4-oxadiazin-5-onderivate der allgemeinen Formel I durch Austausch der Hydroxygruppe gegen ein Halogenatom oder durch O-Acylierung sind, bei welcher es sich um eine an sich bekannte Umsetzung handelt, kann ebenfalls unter den üblichen Reaktionsbedingungen, zum Beispiel durch Umsetzen der ersteren mit Thionylchlorid beziehungsweise mit den einzuführenden Acylrest aufweisenden Acylhalogeniden in Gegenwart von säurebindenden Mitteln, zum Beispiel von Triäthylamin, durchgeführt werden.

Besonders leicht geht der nach der Gegebenenfallsmaßnahme des erfindungsgemäßen Verfahrens erfolgende Austausch des Benzyloxycarbonylrestes in der 2-Stellung gegen einen Acetylrest als Alkylcarbonylrest vor sich. Hierzu werden vorzugsweise die erfindungsgemäßen Tetrahydro-1,2,4-oxadiazin-5-onderivate der allgemeinen Formel I, bei welchen $R_2$ für einen Benzyloxycarbonylrest steht, in Essigsäureanhydrid oder in Gemischen von Essigsäureanhydrid und Dimethylformamid in Gegenwart von Katalysatoren hydriert, wobei mit dem Abspalten des Benzyloxycarbonylrestes gleichzeitig auch der Acetylrest in die 2-Stellung eingeführt wird. Dieser Austausch kann aber auch in 2 getrennten Schritten durchgeführt werden, wobei zuerst der Benzyloxycarbonylrest in der 2-Stellung durch katalytische Hydrierung in einem neutralen Medium abgespalten und dann das Stickstoffatom in der 2-Stellung auf übliche Weise acyliert wird.

Das Abtrennen und Gewinnen beziehungsweise Reinigen des Endproduktes aus dem Reaktionsgemisch können bei

sämtlichen Varianten des erfindungsgemäßen Verfahrens auf die oben bei der Variante a) beschriebene Weise durchgeführt werden.

Die im erfindungsgemäßen Verfahren als Ausgangsverbindungen verwendbaren α-Aminooxycarbonsäureamide der allgemeinen Formel II sind bereits bekannt. Ihre Herstellung wurde zuerst in der ungarischen Patentschrift 162 469 (entspricht der deutschen Patentschrift 2 064 061 beziehungsweise der österreichischen Patentschrift 305 342) beschrieben. Die ferner im erfindungsgemäßen Verfahren als Ausgangsverbindungen verwendbaren Tetrahydro-1,2,4--oxadiazin-5-onderivate der allgemeinen Formel IV sind im Schrifttum bisher nicht beschriebene neue Verbindungen, welche aus den entsprechenden α-Aminooxycarbonsäureamiden, die an Stelle des Restes, für den $R_4$' steht, Wasserstoff aufweisen, durch deren Umsetzen mit gegebenenfalls am Ring durch 1 bis 3 Alkoxyrest(e) mit 1 bis 4 Kohlenstoffatom(en) substituiertem Benzaldehyd hergestellt worden sein können. Diese Umsetzung kann in protonischen oder aprotonischen Medien in Gegenwart von Säuren durchgeführt worden sein. Die näheren Einzelheiten dieses Verfahrens sind in der den ungarischen Patentanmeldungen mit den Aktenzeichen 3163/80 und 3164/80 vom 31. Dezember 1980 entsprechenden gleichzeitig eingereichten anderen europäischen Patentanmeldung derselben Anmelderin beschrieben.

Ferner sind erfindungsgemäß Arzneimittel, welche 1 oder mehr der erfindungsgemäßen Tetrahydro-1,2,4--oxadiazin-5-onderivate der allgemeinen Formel I als Wirkstoff beziehungsweise Wirkstoffe, gegebenenfalls zusammen mit 1 oder mehr pharmazeutisch üblichen festen oder flüssigen Trägerstoff(en) und/oder Hilfsstoff(en), enthalten, vorgesehen.

Die erfindungsgemäßen Tetrahydro-1,2,4-oxadiazin-5-onderivate haben nämlich wie bereits erwähnt wertvolle pharmakologische Wirkungen, insbesondere auf das Zentralnervensystem, vor allem krampfhemmende beziehungsweise antikonvulsive Wirkungen.

Die pharmakologischen Wirkungen der erfindungsgemäßen Tetrahydro-1,2,4-oxadiazin-5-onderivate der allgemeinen Formel I wurden in Tierversuchen nach den üblichen pharmakologischen Verfahrensweisen nachgewiesen, wobei als Vergleichssubstanzen die anerkannt gut wirksamen bekannten Handelsprodukte 5,5-Diphenylhydantoin [Vergleichssubstanz A] und 3-Methyl-5-äthyl-5-phenylhydantoin [Vergleichssubstanz B] gleicher Wirkungsrichtung (krampfhemmende beziehungsweise antikonvulsive und antiepileptische Mittel) verwendet wurden. Die Tierversuche wurden mit CFLP-Mäusen (LATI, Gödöllő, Ungarn) von gemischtem Geschlecht (Körpergewicht: 18 bis 32 g) mit Gruppen von je 10 Tieren durchgeführt. Die zu untersuchenden Verbindungen wurden in einer 5%-igen wäßrigen Lösung von Polyoxyäthylensorbitanmonooleat [Tween 80®] suspendiert und diese Suspensionen wurden auf peroralem Wege mit einer Sonde den Tieren verabreicht. Die Wirkstoffe wurden in den zur Untersuchung der krampfhemmenden beziehungsweise antikonvulsiven Wirkung durchgeführten Versuchen in Dosen von 20 mg/kg Körpergewicht und zur Messung der neurotoxischen Wirkung in Dosen von 80 mg/kg Körpergewicht verabreicht. Die Wirkungen wurden 1 Stunde nach der Verabreichung durch die im folgenden beschriebenen Verfahrensweisen gemessen.

I) Prüfung der krampfhemmenden beziehungsweise antikonvulsiven Wirkung

a) Maximaler Elektroschock [MES]

Die Tiere wurden nach der Verfahrensweise von E. A. Swinyard und Mitarbeitern (J. Pharmacol. 106 [1952], 319) mit einer Hornhautelektrode beziehungsweise Corneal--Elektrode (20 mA, 0,2 Sekunde) geschockt. 100% der Blindversuchs- beziehungsweise Kontrolltiere reagierten auf diesen Reiz mit einem tonischen Streckkrampf (extensorischen Krampf) der hinteren Gliedmaßen. Das Unterbleiben dieser Reaktion wurde als unter der Wirkung der Behandlung eingetretener Schutz gewertet.

b) Hemmwirkung gegen den Pentamethylentetrazolkrampf [Pentetrazolkrampf]

Es wurden 125 mg Pentamethylentetrazol/kg Körpergewicht nach der Verfahrensweise von Cr. N. Everett und R. K. Richards (J. Pharmacol. Exp. Therap. 81 [1944], 402) den Tieren subkutan verabreicht. 1 Stunde nach der Verabreichung des zu untersuchenden Wirkstoffes wurde die Wirkung beobachtet: Als Schutzwirkung wurde das Unterbleiben des klonischen Krampfes beziehungsweise des tonischen Streckkrampfes der hinteren Gliedmaßen bewertet.

## II) Prüfung der neurotoxischen Wirkung

### Messung der Muskelinkoordination [RR] an Mäusen

Die Veränderung der koordinierten Muskelbewegung wurde nach der Verfahrensweise von W. J. Kinnard und C. F. Carr (Brit. J. Pharmacol. 121 [1957], 354) an einem gedrehten Stab (Durchmesser: 20 mm; Frequenz: 12 Umdrehungen/Minute) geprüft. Die normal trainierten Tiere konnten 120 Sekunden lang am gedrehten Stab bleiben. Es wurde 1 Stunde nach der Verabreichung der zu untersuchenden Wirkstoffe beobachtet, wie viele Tiere innerhalb 120 Sekunten vom gedrehten Stab gefallen sind, also bei wieviel Prozent der Tiere eine Muskelinkoordination aufgetreten ist.

## III) Akute Toxizität

Die Toxizität der zu untersuchenden Verbindungen wurde nach 1-maliger Verabreichung von verschiedenen Dosen durch 14 Tage lange Beobachtung der Tiere ermittelt. Die $LD_{50}$-Werte (die bei 50% der Tiere tödlichen Dosen) wurden auf Grund des Prozentsatzes der innerhalb 14 Tage verendeten Tiere berechnet.

Die erhaltenen Ergebnisse sind in der folgenden Tabelle 1 zusammengestellt.

Tabelle 1

| Verbindung Bei-spiel-Nr. | Symbole $R_2$ | $R_3$ | $R_4$ | Hemmwirkung in % in Bezug auf den maxi-malen Elektro-schock | Pentamethylen-tetrazolkrampf tonischen Streckkrampf | klonischen Krampf | Muskel-inkoor-dina-tion in % | Peroraler $LD_{50}$-Wert in mg/kg |
|---|---|---|---|---|---|---|---|---|
| 10 | Acetyl | Phenyl | Methyl | 40 | 20 | - | 20 | 800 |
| 4 | Acetyl | Phenyl | Äthyl | - | 20 | - | 20 | 800 |
| 14 | Acetyl | Phenyl | n-Hexyl | - | 40 | - | - | 800 |
| 5 | Acetyl | Phenyl | Hydroxymethyl | 60 | 40 | - | 20 | 800 |
| 7 | Acetyl | Phenyl | Chlormethyl | 40 | - | - | 40 | 800 |
| 6 | Acetyl | Phenyl | Acetoxymethyl | 70 | 60 | - | 40 | 800 |
| 18 | Acetyl | Phenyl | Benzoyloxymethyl | 40 | 70 | - | 40 | 800 |

Fortsetzung der Tabelle 1

| Verbindung: Beispiel-Nr. | Symbole: $R_2$ | Symbole: $R_3$ | Symbole: $R_4$ | Hemmwirkung in % in Bezug auf den maximalen Elektroschock | Hemmwirkung in % in Bezug auf den Pentamethylentetrazolkrampf: tonischen Streckkrampf | Hemmwirkung in % in Bezug auf den Pentamethylentetrazolkrampf: klonischen Krampf | Muskelinkoordination in % | Peroraler $LD_{50}$-Wert in mg/kg |
|---|---|---|---|---|---|---|---|---|
| 19 | Acetyl | Phenyl | 3,4,5-Trimethoxybenzoyloxymethyl | 30 | - | - | - | 800 |
| Vergleichssubstanz A | | | Diphenylhydantoin | 90 | 80 | - | 70 | 279 |
| Vergleichssubstanz B | | | 3-Methyl-5-äthyl-5-phenylhydantoin | 10 | 50 | - | 50 | 476 |

Aus den Daten der obigen Tabelle 1 geht hervor, daß die erfindungsgemäßen Tetrahydro-1,2,4-oxadiazin-5-onderivate der allgemeinen Formel 1 überlegene krampfhemmende beziehungsweise antikonvulsive Wirkungen haben, wobei ihre neurotoxische Wirkung unbedeutend ist (geringere Werte der Muskelinkoordination als bei den Vergleichssubstanzen) und mit ihnen tödliche Wirkungen nur bei sehr hohen Dosen auftreten (höhere $LD_{50}$-Werte als die der Vergleichssubstanzen), so daß sie bei höheren bis gleichen therapeutischen Indices eine wesentlich größere therapeutische Breite haben als die bekannten Verbindungen gleicher Wirkungsrichtung. Auf Grund dieser vorteilhaften Eigenschaften können die erfindungsgemäßen Tetrahydro-1,2,4-oxadiazin-5-onderivate der allgemeinen Formel I vorteilhafter als die auf diesem Gebiet in weitem Umfang verwendeten bekannten Hydantoinderivate zur Behandlung von epileptischen Erkrankungen eingesetzt werden.

Die erfindungsgemäßen Tetrahydro-1,2,4-oxadiazin-5-onderivate können in der Therapie in Form von üblichen Arzneimittelpräparaten verwendet werden. Solche Arzneimittelpräparate können das beziehungsweise die erfindungsgemäße(n) Tetrahydro-1,2,4-oxadiazin-5-onderivat(e) zusammen mit 1 oder mehr an sich bekannten zur enteralen oder parenteralen Verabreichung geeigneten festen oder flüssigen anorganischen oder organischen Trägerstoff(en) und/oder Hilfsstoff(en) enthalten. Sie können beispielsweise in Form von Tabletten, Injektionslösungen, verdünnten oder konzentrierten Suspensionen oder Emulsionen oder Zäpfchen zubereitet sein.

Diese Arzneimittelpräparate können in einer Dosierungseinheit (wie Tablette oder Ampulle) zweckmäßig Mengen von

30 bis 100 mg des beziehungsweise der jeweiligen erfindungsgemäßen Tetrahydro-1,2,4-oxadiazin-5-onderivate[s] der allgemeinen Formel I als Wirkstoff(e) enthalten.

Die erfindungsgemäßen Arzneimittel können in der Humantherapie peroral oder parenteral, insbesondere intravenös, verabreicht werden. Die tägliche Dosis der Wirkstoffe Tetrahydro-1,2,4-oxadiazin-5-onderivate kann bei erwachsenen Menschen unter Berücksichtigung der Art der Krankheit, des Zustandes der Patienten und der gewünschten Wirkung im allgemeinen 200 bis 600 mg betragen, wobei diese Tagesdosen auf einmal oder während des Tages auf mehrere kleinere Dosen verteilt verabreicht werden können.

Die Erfindung wird an Hand der folgenden Beispiele näher erläutert.

Die in den Beispielen angegebenen Schmelzpunkte wurden in einem Schmelzpunktmeßgerät nach Dr. Tottoli [Büchi] bestimmt. Die Dünnschichtchromatogramme wurden auf gegen UV-Licht sensibilisierten Silicagelplatten ("Kieselgel G® nach Stahl" [Merck]) hergestellt. Zur Entwicklung der Chromatogramme wurde ein Gemisch von Benzol und Aceton im Volumverhältnis von 1 : 1 verwendet.

Die Entwicklung der Dünnschichtchromatogramme wurde in der Mehrzahl der Fälle nach einer der folgenden Verfahrensweisen oder Kombinationen derselben durchgeführt:

I. UV-Bestrahlung (Wellenlänge: 254 nm).

II. Behandlung mit Joddämpfen.

III. Chlorierung und nachfolgendes Besprühen mit Tolidin/Kaliumjodid.

Die Identifizierung der Struktur der hergestellten Verbindungen erfolgte auf Grund der Elementaranalysen, der Ultrarotspektren (IR-Spektren) und der magnetischen Kernresonanzspektren (NMR-Spektren) der Verbindungen. Die Ultrarotspektren wurden in einem Apparat vom Typ "Perkin-Elmer 257" und die magnetischen Kernresonanzspektren in einem magnetischen Kernresonanzspektrometer vom Typ "Varion EM-60" aufgenommen.

Bei der Aufarbeitung der Reaktionsgemische wurden die Lösungen der erhaltenen Produkte in einem "Rotavapor R" [Büchi] Vakuumeindampfer vom Typ "Rotavapor R" [Büchi] bei 50°C nicht übersteigender Temperaturen eingedampft.



BAD ORIGINAL

Beispiel 1

2-Benzyloxycarbonyl-3-phenyl-4-methyl-tetrahydro--1,2,4-oxadiazin-5-on

nach der Variante a)
[Verfahrensweise A]

Das Gemisch von 2,29 g (9,6 mMol) N-(Benzyloxycarbonyl)-aminooxy-N'-methyl-acetamid, 2,0 ml (19,8 mMol) Benzaldehyd und 0,3 g Camphersulfonsäure wurde in 40 ml Benzol unter einem mit einem Marcusson'schen Wasserabscheider-Aufsatz ausgestatteten Rückflußkühler 3 Tage lang gekocht; das während der Reaktion entstandene Wasser wurde laufend aus dem Reaktionsgemisch entfernt. Das Fortschreiten der Reaktion wurde durch Dünnschichtchromatographie kontrolliert und nach der Beendigung der Reaktion wurde das Reaktionsgemisch zur Trockne eingedampft. Der Rückstand wurde aus dem Gemisch von 5 ml Äther und 20 ml Hexan kristallisiert; es wurden auf diese Weise 1,65 g 2-Benzyloxycarbonyl-3-phenyl--4-methyltetrahydro-1,2,4-oxadiazin-5-on (50 % d. Th.) erhalten; F. 82-83 °; $R_f$ = 0,7.

Analyse für $C_{18}H_{18}N_2O_4$ (Mol. Gew.: 326,35):
berechnet: C 66,25 %, H 5,56 %, N 8,58 %;
gefunden: C 66,27 %, H 5,30 %, N 8,52 %.

IR (KBr): 1730 (>C=O), 1665 (>C=O, Amid), 1410 (Ring), 745, 700 (aromatisch) $cm^{-1}$.

NMR (DMSO-$d_6$, $CDCl_3$, TMS) ppm: 2,83 s ($-CH_3$), 4,58 s ($-CH_2-C=O$), 5,28 s ($-CH_2-C_6H_5$), 6,49 s (>CH-), 7,25 - 8,6 m (10H, aromatisch).

Beispiel 2

2-Benzyloxycarbonyl-3-phenyl-4-methyl-tetrahydro-1,2,4-oxadiazin-5-on

nach der Variante b)
[Verfahrensweise B]

Die Suspension von 0,24 g (5 mMol) 50 %igem Natriumhydrid in 10 ml wasserfreiem Tetrahydrofuran wurde unter lebhaftem Rühren mit der Lösung von 1,56 g (5 mMol) 2-Benzyloxycarbonyl-3-phenyl-tetrahydro-1,2,4-oxadiazin-5-on in 10 ml wasserfreiem Tetrahydrofuran versetzt. Die erhaltene Suspension wurde bei 0 °C 20 Minuten gerührt, dann wurde die Lösung von 0,35 ml (5,6 mMol) Methyljodid in 5 ml Tetrahydrofuran tropfenweise zugesetzt und das Gemisch über Nacht bei Raumtemperatur gerührt. Am nächsten Tag wurde das Reaktionsgemisch mit einigen Tropfen Essigsäure versetzt und das Lösungsmittel wurde unter vermindertem Druck verdampft. Der Rückstand wurde in 10 ml Chloroform gelöst, die Lösung mit 3 ml Wasser, dann mit 3 ml n Salzsäurelösung und zuletzt mit 3 ml n Natriumbicarbonatlösung ausgeschüttelt, mit wasserfreiem Natriumsulfat getrocknet und eingedampft. Der Rückstand wurde aus dem Gemisch von 2 ml Isopropanol und 5 ml Hexan kristallisiert; es wurden auf diese Weise 0,25 g 2-Benzyloxycarbonyl-3-phenyl-4-methyl-tetrahydro-1,2,4-oxadiazin-5-on (15 % d. Th.) erhalten; F. 82-83 °C; $R_f$ = 0,7. Auch die übrigen physikalisch-chemischen Konstanten des Produkts sind mit jenen der nach Beispiel 1 erhaltenen Verbindung identisch.

Beispiel 3

2-Benzyloxycarbonyl-3-phenyl-4-äthyl-tetrahydro-
-1,2,4-oxadiazin-5-on

nach der Variante a)
[Verfahrensweise C]

0,65 g (2,5 mMol) N-(Benzyloxycarbonyl)-aminooxy-N'-äthyl-acetamid und 0,4 ml (4 mMol) Benzaldehyd wurden im Gemisch von 2,5 ml Essigsäure und 0,3 ml Essigsäureanhydrid gelöst, die Lösung wurde mit 0,12 ml konz. Schwefelsäure versetzt und eine Stunde bei Raumtemperatur stehengelassen. Dann wurden 0,7 g Natriumacetat-trihydrat zugesetzt und das Gemisch wurde im Vakuum zur Trockne eingedampft. Der Rückstand wurde in 10 ml Äthylacetat gelöst, die Lösung wurde mit 3 ml einer wäßrigen n Natriumbicarbonatlösung, dann mit 3 ml n Salzsäure und schließlich zweimal mit je 3 ml Wasser ausgeschüttelt, mit wasserfreiem Natriumsulfat getrocknet und zur Trockne eingedampft. Als Rückstand wurden 0,69 g 2-Benzyloxycarbonyl-3-phenyl-4-äthyl-tetrahydro-1,2,4-oxadiazin-5-on (75 % d. Th.) in der Form eines chromatographisch beinahe vollkommen reinen öligen Produkts erhalten; $R_f$ = 0,8. Dieses Produkt kann ohne weitere Reinigung als Ausgangsverbindung der im Beispiel 4 beschriebenen weiteren Reaktion eingesetzt werden.



BAD ORIGINAL

Beispiel 4

2-Acetyl-3-phenyl-4-äthyl-tetrahydro-1,2,4-oxadiazin-5-on

nach der Gegebenenfallsmaßnahme [Verfahrensweise D]

0,69 g (2 mMol) 2-Benzyloxycarbonyl-3-phenyl-4-äthyl-tetrahydro-1,2,4-oxadiazin-5-on wurden in 7 ml Essigsäureanhydrid gelöst und nach der Zugabe von 0,1 g 5 %igem Palladium/Aktivkohle-Katalysator wurde bei Raumtemperatur Wasserstoffgas unter lebhaftem Rühren durch das Gemisch geleitet. Das Fortschreiten der Hydrierungsreaktion wurde durch Dünnschichtchromatographie kontrolliert; nach der Beendigung der Reaktion wurde der Katalysator abfiltriert, das Filtrat wurde unter vermindertem Druck zur Trockne eingedampft und der Rückstand wurde aus 1 ml Äthanol umkristallisiert. Auf diese Weise wurden 0,41 g 2-Acetyl-3-phenyl-4-äthyl-tetrahydro-1,2,4-oxadiazin-5-on (82 % d. Th.) erhalten; F. 130-132 °C; $R_f$ = 0,6.

Analyse für $C_{13}H_{16}N_2O_3$ (Mol. Gew.: 248,26):

berechnet: C 62,88 %, H 6,49 %, N 11,27 %;

gefunden: C 62,25 %, H 6,37 %, N 11,12 %.

IR (KBr): 1645 (>C=O), 1274 (tertiäres Amid), 740, 693 (aromatisch) $cm^{-1}$.

NMR ($CDCl_3$, TMS) ppm: 1,16 t ($-CH_2-\underline{CH_3}$), 2,13 s ($CH_3CO-$), 2,86 und 3,9 m ($>N-\underline{CH_2}-CH_3$), 4,6 s ($-CH_2-CO-$), 6,7 s (>CH-), 7,40 s (5H, aromatisch).

Beispiel 5

2-Acetyl-3-phenyl-4-hydroxymethyl-tetrahydro-
-1,2,4-oxadiazin-5-on

nach der Variante c), 1. Stufe

Die Lösung von 1,1 g (5 mMol) 2-Acetyl-3-phenyl-tetrahydro-1,2,4-oxadiazin-5-on in 10 ml Tetrahydrofuran wurde mit 1 ml Triäthylamin und 0,4 ml 37 %-iger wäßriger Formaldehydlösung versetzt. Das Gemisch wurde bei Raumtemperatur über Nacht stehengelassen und dann zur Trockne eingedampft. Der Rückstand wurde mit Äther verrieben und abfiltriert. Auf diese Weise wurden 1,16 g 2-Acetyl-3-phenyl-4-hydroxymethyl-tetrahydro-1,2,4-oxadiazin-5-on (93 % d. Th.) erhalten; F.123-126 °C; $R_f$ = 0,5.

Analyse für $C_{12}H_{14}N_2O_4$ (Mol. Gew.: 250,25):
berechnet: C 57,59 %, H 5,64 %, N 11,19 %;
gefunden: C 57,49 %, H 6,03 %, N 10,99 %.
IR (KBr): 3330, 1053 (-OH), 1675 (>C=O), 1648 (>C=O, Amid), 753, 710 (aromatisch) $cm^{-1}$.
NMR (DMSO-$d_6$ + $CDCl_3$, TMS) ppm: 2,0 s (-$CH_3$), 4,7 AB q (-$CH_2$-CO-), 4,1 2xd und 5,5 2xd (>N-$CH_2$OH), 6,0 t (-OH), 7,0 s (>CH-), 7,5 s (5H, aromatisch).

- 26 -

Beispiel 6

2-Acetyl-3-phenyl-4-acetoxymethyl-tetrahydro-1,2,4-oxadiazin-5-on

nach der Variante c), 2. Stufe
[Verfahrensweise E]

Die Lösung von 1,0 g (4 mMol) 2-Acetyl-3-phenyl-4-hydroxymethyl-tetrahydro-1,2,4-oxadiazin-5-on in 10 ml wasserfreiem Tetrahydrofuran wurde mit 0,72 ml wasserfreiem Triäthylamin versetzt. Die erhaltene Lösung wurde auf 0 °C gekühlt und bei dieser Temperatur wurden 0,36 ml (0,40 g, 5 mMol) Acetylchlorid tropfenweise zugesetzt. Das Gemisch wurde dann bei Raumtemperatur 2 Stunden gerührt, anschließend wurde das gefällte Triäthylamin-hydrochlorid abfiltriert und das Filtrat zur Trockne eingedampft. Der Rückstand wurde in 10 ml Äthylacetat gelöst, die Lösung mit 3 ml n Natriumbicarbonatlösung, dann mit 3 ml n Salzsäure und schließlich zweimal mit je 3 ml Wasser ausgeschüttelt, mit wasserfreiem Natriumsulfat getrocknet und zur Trockne eingedampft. Der erhaltene Rückstand wurde aus dem Gemisch von 3 ml Äther und 6 ml n-Hexan umkristallisiert; auf diese Weise wurden 0,90 g 2-Acetyl-3-phenyl-4-acetoxymethyl-tetrahydro-1,2,4-oxadiazin-5-on (77 % d. Th.) erhalten; F. 75-76 °C; $R_f$ = 0,7.

Analyse für $C_{14}H_{16}N_2O_5$ (Mol. Gew.: 292,28):
berechnet: C 57,52 %, H 5,52 %, N 9,58 %;
gefunden: C 57,30 %, H 5,56 %, N 9,49 %.

IR (KBr): 1740 (>C=O, Ester), 1680 (>C=O, Amid), 1210 (>C-O-C<), 750-700 (aromatisch) $cm^{-1}$.

NMR ($DMSO-d_6$, TMS) ppm: 2,0 s ($-CH_3$), 2,13 s ($-CH_3$), 4,66 s ($-CH_2-CO-$), 4,96 und 5,46 AB q (>$N-CH_2-$), 6,9 s (>CH-), 7,46 (5H, aromatisch).

Beispiel 7

2-Acetyl-3-phenyl-4-chlormethyl-tetrahydro-1,2,4-oxadiazin-5-on

nach der Variante c), 2. Stufe

0,5 g (2 mMol) 2-Acetyl-3-phenyl-4-hydroxymethyl-tetrahydro-1,2,4-oxadiazin-5-on wurden in 3 ml Thionylchlorid eine Stunde unter Rückfluß gekocht. Die erhaltene Lösung wurde unter vermindertem Druck zur Trockne eingedampft, der Rückstand wurde mit auf 0 °C abgekühltem Äther verrieben und abfiltriert. Das erhaltene Produkt wurde aus dem Gemisch von 2 ml Chloroform und 6 ml n-Hexan umkristallisiert; auf diese Weise wird 0,28 g 2-Acetyl-3-phenyl-4-chlormethyl-tetrahydro-1,2,4-oxadiazin-5-on (53 % d. Th.) erhalten; F. 171-172 °C; $R_f$ = 0,7

Analyse für $C_{12}H_{13}N_2O_3$ (Mol. Gew.: 268,70):

berechnet: C 53,64 %, H 4,88 %, N 10,43 %, Cl 13,20 %;
gefunden: C 53,10 %, H 4,78 %, N 9,77 %, Cl 13,57 %.

IR (KBr): 1683, 1665 (>C=O), 753, 698 (aromatisch), 680 (>C-Cl) $cm^{-1}$.

NMR ($CDCl_3$, TMS) ppm: 2,13 s ($-CH_3$), 4,67 s ($-CH_2$-CO-), 4,41 und 6,91 AB q (>N-$CH_2$Cl), 6,93 s (-CH-), 7,46 s (5H, aromatisch).

Nach den in den obigen Beispielen beschriebenen Verfahrensweisen A bis E wurden auch die in der folgenden Tabelle 2 aufgezählten weiteren erfindungsgemäßen Tetrahydro-1,2,4-oxadiazin-5-onderivate der allgemeinen Formel I (Beispiele 8 bis 19) hergestellt. In dieser Tabelle 2 sind die einzelnen Verbindungen durch die Angabe der Symbole

$R_2$ , $R_3$ und $R_4$ definiert. In dieser Tabelle 2 sind neben den in den einzelnen Fällen verwendeten Herstellungsverfahrensweisen auch die Schmelzpunkte und $R_f$-Werte der einzelnen Verbindungen, die Ausbeuten an ihnen sowie die beim Umkristallisieren der Produkte verwendeten Lösungsmittel beziehungsweise Lösungsmittelgemische angegeben.

Tabelle 2

| Verbindung Beispiel Nr. | Symbole $R_2$ | $R_3$ | $R_4$ | Schmelzpunkt in °C | $R_f$-Wert | Verfahrensweise | Ausbeute in % der Theorie | Umkristallisationslösungsmittel |
|---|---|---|---|---|---|---|---|---|
| 8 | Benzyloxycarbonyl | 2,5-Dimethoxyphenyl | Methyl | 98 bis 100 | 0,8 | B | 25 | Äthanol |
| 9 | Benzyloxycarbonyl | Phenyl | Methyl | 82 bis 83 | 0,7 | B*) | 50 | Gemisch von Äther und n-Hexan im Volumverhältnis von 1 : 4 |
| 10 | Acetyl | Phenyl | Methyl | 100 bis 101 | 0,5 | B | 20 | - |
| 11 | Acetyl | Phenyl | n-Propyl | 74 bis 76 | 0,6 | A bzw. B | 45 | - |
| 12 | Acetyl | Phenyl | Isopropyl | 102 | 0,6 | C bzw. D | 39 | Äthanol |
| 13 | Acetyl | Phenyl | Cyclopropyl | 165 | 0,6 | A bzw. D | 30 | Äthanol |
| 14 | Acetyl | Phenyl | n-Hexyl | Öl | 0,7 | A bzw. D | 32 | - |

Fortsetzung der Tabelle 2

| Verbindung Beispiel Nr. | Symbole $R_2$ | $R_3$ | $R_4$ | Schmelzpunkt in °C | $R_f$-Wert | Verfahrensweise | Ausbeute in % der Theorie | Umkristallisationslösungsmittel |
|---|---|---|---|---|---|---|---|---|
| 15 | Acetyl | Phenyl | Cyclohexyl | 124 bis 126 | 0,6 | A bzw. D | 38 | Gemisch von Äthylacetat und n-Hexan im Volumverhältnis von 1 : 3 |
| 16 | Acetyl | Phenyl | n-Octyl | Öl | 0,7 | C bzw. D | 45 | - |
| 17 | Acetyl | Phenyl | n-Undecyl | Öl | 0,8 | A bzw. D | 41 | - |
| 18 | Acetyl | Phenyl | Benzoyloxymethyl | 83 bis 84 | 0,7 | E | 75 | Gemisch von Äther und n-Hexan im Volumverhältnis von 1 : 2 |
| 19 | Acetyl | Phenyl | 3,4,5-Trimethoxybenzoyloxymethyl | 150 bis 151 | 0,7 | E | 72 | Äthanol |

*) jedoch Aufarbeiten durch Säulenchromatographie auf Silicagel mit einem Gemisch von Benzol und Aceton im Volumverhältnis von 1 : 1

Patentansprüche

Patentansprüche

1.) Tetrahydro-1,2,4-oxadiazin-5-onderivate der allgemeinen Formel

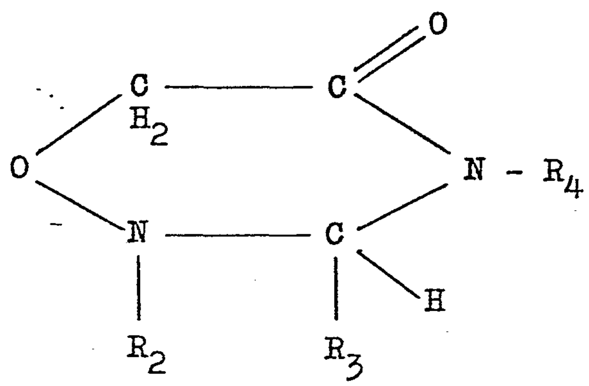

I ,

worin

$R_2$ für einen Benzyloxycarbonylrest oder einen Alkylcarbonylrest mit 1 bis 4 Kohlenstoffatom(en) im Alkylteil steht,

$R_3$ einen, gegebenenfalls durch 1 bis 3 Alkoxyrest(e) mit 1 bis 4 Kohlenstoffatom(en) substituierten, Phenylrest bedeutet und

$R_4$ einen Alkyl- oder Cycloalkylrest mit 1 bis 11 Kohlenstoffatom(en), einen Hydroxymethylrest, einen Halogenmethylrest, einen Alkylcarbonyloxymethylrest mit 1 bis 4 Kohlenstoffatom(en) im Alkylteil oder einen, gegebenenfalls durch 1 bis 3 Alkoxyrest(e) mit 1 bis 4 Kohlenstoffatom(en) substituierten, Benzoyloxymethylrest darstellt.

- 32 -

2.) Tetrahydro-1,2,4-oxadiazin-5-onderivate nach Anspruch 1, dadurch gekennzeichnet, daß der Alkylcarbonylrest, für den $R_2$ stehen kann, ein solcher mit 1 oder 2 Kohlenstoffatom(en) im Alkylteil, insbesondere ein Acetylrest, ist.

3.) Tetrahydro-1,2,4-oxadiazin-5-onderivate nach Anspruch 1 oder 2, dadurch gekennzeichnet, daß der beziehungsweise die Alkoxyrest(e), durch welchen beziehungsweise welche der Phenylrest, für den $R_3$ stehen kann, substituiert sein kann, ein solcher beziehungsweise solche mit 1 oder 2, insbesondere 1, Kohlenstoffatom(en) ist beziehungsweise sind.

4.) Tetrahydro-1,2,4-oxadiazin-5-onderivate nach Anspruch 1 bis 3, dadurch gekennzeichnet, daß der Alkylrest, für den $R_4$ stehen kann, ein solcher mit 1 bis 8, insbesondere 1 bis 6, Kohlenstoffatom(en) ist.

5.) Tetrahydro-1,2,4-oxadiazin-5-onderivate nach Anspruch 1 bis 3, dadurch gekennzeichnet, daß der Cycloalkylrest, für den $R_4$ stehen kann, ein solcher mit 3 bis 8, insbesondere 3 bis 6, Kohlenstoffatomen ist.

6.) Tetrahydro-1,2,4-oxadiazin-5-onderivate nach Anspruch 1 bis 3, dadurch gekennzeichnet, daß der Halogenmethylrest, für den $R_4$ stehen kann, ein Chlormethyl- oder Brommethylrest ist.

7.) Tetrahydro-1,2,4-oxadiazin-5-onderivate nach Anspruch 1 bis 3, dadurch gekennzeichnet, daß der Alkylcarbonyloxymethylrest, für den $R_4$ stehen kann,

ein solcher mit 1 oder 2, insbesondere 1, Kohlenstoffatom(en) im Alkylteil ist.

8.) Tetrahydro-1,2,4-oxadiazin-5-onderivate nach Anspruch 1 bis 3, dadurch gekennzeichnet, daß der beziehungsweise die Alkoxyrest(e), durch welchen beziehungsweise welche der Benzoyloxymethylrest, für den $R_4$ stehen kann, substituiert sein kann, ein solcher beziehungsweise solche mit 1 oder 2, insbesondere 1, Kohlenstoffatom(en) ist beziehungsweise sind.

9.) 2-Acetyl-3-phenyl-4-(hydroxymethyl)-tetrahydro-1,2,4-oxadiazin-5-on.

10.) 2-Acetyl-3-phenyl-4-(acetoxymethyl)-tetrahydro-1,2,4-oxadiazin-5-on.

11.) 2-Acetyl-3-phenyl-4-(benzoyloxymethyl)-tetrahydro-1,2,4-oxadiazin-5-on.

12.) Verfahren zur Herstellung der Verbindungen dadurch gekennzeichnet, daß man

a) zur Herstellung von Tetrahydro-1,2,4-oxadiazin-5-onderivaten der allgemeinen Formel I, bei welchen $R_4$ einen Alkyl- oder Cycloalkylrest mit 1 bis 11 Kohlenstoffatom(en) darstellt, α-Aminooxycarbonsäureamide der allgemeinen Formel

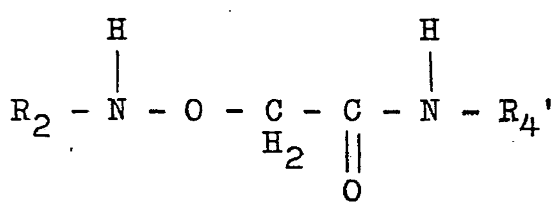

II ,

worin

$R_4'$' einen Alkyl- oder Cycloalkylrest mit 1 bis 11 Kohlenstoffatom(en) bedeutet und

$R_2$ wie im Anspruch 1 oder 2 festgelegt ist,

in aprotonischen oder protonischen Medien in Gegenwart von Säuren mit, gegebenenfalls am Ring durch 1 bis 3 Alkoxyrest(e) mit 1 bis 4 Kohlenstoffatom(en) substituiertem, Benzaldehyd der allgemeinen Formel

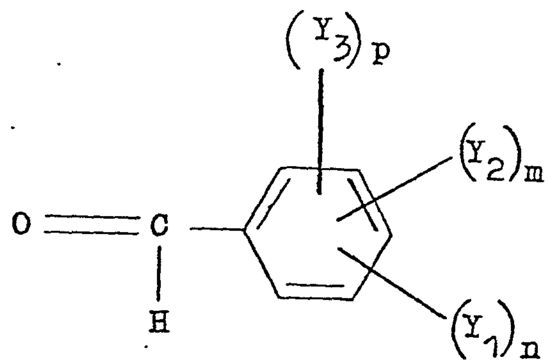

III ,

worin

$Y_1$ , $Y_2$ und $Y_3$ für Alkoxyreste mit 1 bis 4 Kohlenstoffatom(en) stehen und

n, m und p 0 oder 1 sind,

kondensiert oder

b) zur Herstellung der Tetrahydro-1,2,4-oxadiazin-5-onderivate der allgemeinen Formel I, bei wel-

chen $R_4$ einen Alkyl- oder Cycloalkylrest mit 1 bis 11 Kohlenstoffatom(en) bedeutet, Tetrahydro-1,2,4-oxadiazin-5-onderivate der allgemeinen Formel

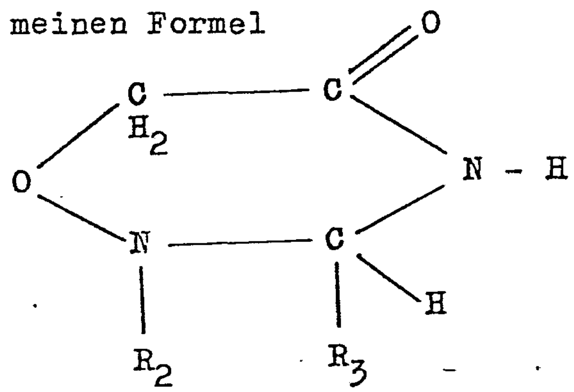

IV ,

worin $R_2$ und $R_3$ wie in den Ansprüchen 1 bis 3 festgelegt sind, in Gegenwart von Basen mit Verbindungen der allgemeinen Formel

$$X - R_4' \qquad V ,$$

worin

X für einen reaktionsfähigen Esterrest, insbesondere ein Halogenatom, steht und

$R_4'$ wie oben festgelegt ist,

umsetzt oder

c) zur Herstellung der Tetrahydro-1,2,4-oxadiazin--5-onderivate der allgemeinen Formel I, bei welchen $R_4$ für einen Hydroxymethylrest, einen Halogenmethylrest, einen Alkylcarbonyloxymethylrest mit 1 bis 4 Kohlenstoffatom(en) im Alkylteil oder einen, gegebenenfalls durch 1 bis 3 Alkoxyrest(e) mit 1 bis 4 Kohlenstoffatom(en) substituierten, Benzoyloxymethylrest steht, Tetrahydro-1,2,4-oxadiazin-5-onderivate der allgemeinen Formel

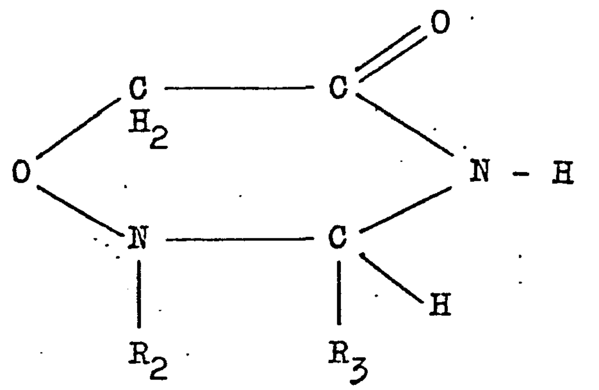

IV ,

worin $R_2$ und $R_3$ wie oben festgelegt sind, in Gegenwart von Basen, insbesondere tertiären organischen Basen mit Formaldehyd umsetzt und gegebenenfalls die erhaltenen Tetrahydro-1,2,4-oxadiazin-5-onderivate der allgemeinen Formel I, bei welchen $R_4$ für einen Hydroxymethylrest steht, durch Behandeln mit Halogenierungsmitteln, insbesondere Thionylhalogeniden, beziehungsweise passenden Acylierungsmitteln, insbesondere Carbonsäurehalogeniden oder Carbonsäureanhydriden, in die entsprechenden Tetrahydro-1,2,4-oxadiazin-5-onderivate der allgemeinen Formel I, bei welchen $R_4$ für einen Halogenmethylrest beziehungsweise einen Alkylcarbonyloxymethylrest mit 1 bis 4 Kohlenstoffatom(en) im Alkylteil oder einen, gegebenenfalls durch 1 bis 3 Alkoxyrest(e) mit 1 bis 4 Kohlenstoffatom(en) substituierten, Benzoyloxymethylrest steht, überführt,

worauf man gegebenenfalls nach einer der obigen Varianten erhaltene Tetrahydro-1,2,4-oxadiazin-5-onderivate der allgemeinen Formel I, bei welchen

$R_2$ für einen Benzyloxycarbonylrest steht, zweckmäßig durch deren katalytisches Hydrieren und Behandeln mit den passenden Acylierungsmitteln, in die entsprechenden Tetrahydro-1,2,4-oxadiazin-5-onderivate der allgemeinen Formel I, bei welchen $R_2$ für einen Alkylcarbonylrest mit 1 bis 4 Kohlenstoffatom(en) im Alkylteil steht, überführt.

13.) Verfahren nach Anspruch 12a), dadurch gekennzeichnet, daß man bei der Umsetzung der α-Aminooxycarbonsäureamide der allgemeinen Formel II mit dem, gegebenenfalls am Ring durch 1 bis 3 Alkoxyrest(e) mit 1 bis 4 Kohlenstoffatom(en) substituierten, Benzaldehyd der allgemeinen Formel III als aprotonische Medien aromatische Kohlenwasserstoffe insbesondere Benzol und/oder Toluol, gegebenenfalls mit einem Zusatz von Butylacetat, verwendet.

14.) Verfahren nach Anspruch 12a), dadurch gekennzeichnet, daß man bei der Umsetzung der α-Aminooxycarbonsäureamide der allgemeinen Formel II mit dem, gegebenenfalls am Ring durch 1 bis 3 Alkoxyrest(e) mit 1 bis 4 Kohlenstoffatom(en) substituierten, Benzaldehyd der allgemeinen Formel III als protonische Medien Gemische von Essigsäure und Essigsäureanhydrid verwendet.

15.) Verfahren nach Anspruch 12a), dadurch gekennzeichnet, daß man bei der Umsetzung der α-Aminooxycarbonsäureamide der allgemeinen Formel II mit dem, gegebenenfalls am Ring durch 1 bis 3 Alkoxyrest(e) mit 1 bis 4 Kohlenstoffatom(en) substituierten, Benzaldehyd der allgemeinen Formel III als Säure Campher-10-sulfonsäure verwendet.

16.) Arzneimittel, gekennzeichnet durch einen Gehalt an 1 oder mehr Verbindung(en) nach Anspruch 1 bis 11 als Wirkstoff(en), gegebenenfalls zusammen mit 1 oder mehr pharmazeutisch üblichen festen oder flüssigen Trägerstoff(en) und/oder Hilfsstoff(en).

Zusammenfassung

0055484

Nummer der Anmeldung

Europäisches Patentamt

# EUROPÄISCHER RECHERCHENBERICHT

EP 81 11 0821.6

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D,A | TETRAHEDRON, Band 35, Nr. 11, 1979<br>Oxford, New York, Toronto<br>F.G. RIDDELL et al. "The Synthesis and Conformational Analysis of Tetrahydro-1,2,4-oxadiazines"<br>Seiten 1391 bis 1398<br>* Seite 1392, Schema *<br>-- | 12 | C 07 D 273/04<br>A 61 K 31/535 |
| A | DE - A1 - 2 640 464 (PHILAGRO S.A.)<br>---- | | |

RECHERCHIERTE SACHGEBIETE (Int. Cl.³)

A 61 K 31/535
C 07 D 273/04

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 16-03-1982 | FROELICH |